# EUROPÄISCHE PATENTANMELDUNG

(11) **EP 2 704 135 A1**
(43) Veröffentlichungstag der Anmeldung: **05.03.2014**
(21) Anmeldenummer: 13004076.9
(22) Anmeldetag: 16.08.2013
(51) Int. Cl.: G10D 1/00, A61H 23/00, A61M 21/00

(54) **Vorrichtung zum Erzeugen von Schallwellen in Wasser**

(30) Priorität: 29.08.2012 DE 102012107991
(71) Anmelder: Bartek, Christian, 82393 Pähl (DE)
(72) Erfinder: Bartek, Christian, 82393 Pähl (DE)
(74) Vertreter: Schlimme, Wolfram

(57) **Zusammenfassung**

Eine Vorrichtung zum Erzeugen von Schallwellen in Wasser, insbesondere zum Erzeugen von multimedial auf einen in einem Wasservolumen befindlichen menschlichen oder tierischen Körper (P) einwirkenden Schallwellen, mit zumindest einem im Wasser schwimmenden Klangkörper (1, 1', 1 ", 101, 101', 201, 301), zeichnet sich dadurch aus, dass der schwimmende Klangkörper (1, 1', 1 ", 101, 101', 201, 301) als Musikinstrument ausgebildet oder der Teil eines Musikinstruments ist und einen Resonanzkörper (10, 10', 10", 110, 110', 210, 310) aufweist, der an zumindest einer Seite mit dem Wasservolumen (2) zur Schalleinleitung in das Wasser in Kontakt steht, worin der Resonanzkörper (10, 10', 10", 110, 110', 210, 310) einen Luftresonanzraum (R, R') aufweist, und dass der Klangkörper (1, 1', 1 ", 101, 101', 201, 301) mit Saiten (16, 116, 116', 216, 316) versehen ist, die bei Anregung den Resonanzkörper (10, 10', 10", 110, 110', 210, 310) und/oder die Luft im Luftresonanzraum (R, R') in Schwingung versetzen.

## Beschreibung

Die vorliegende Erfindung betrifft eine Vorrichtung zum Erzeugen von Schallwellen in Wasser nach dem Oberbegriff des Patentanspruchs 1. Insbesondere betrifft sie eine Vorrichtung zum Erzeugen von multimedial auf einen in einem Wasservolumen befindlichen menschlichen oder tierischen Körper einwirkenden Schallwellen.

### HINTERGRUND DER ERFINDUNG

Schalltherapie ist eine nicht-medizinisch-therapeutische alternative Behandlungsmethode, mit der eine entspannende Wirkung auf einen Patienten erzielt werden soll. Sie wird auch im Wellnessbereich zur allgemeinen Entspannung von Geist und Körper eines Patienten eingesetzt.

Hinreichend bekannt ist die akustische Schalltherapie, mit der beispielsweise entspannende Musik dargeboten wird, die der oder die Patient(en) hören. Es ist auch möglich, die Schallwellen als Körperschall direkt über die Haut auf den Patienten einwirken zu lassen, beispielsweise mittels schwingender Klangschalen, mit denen der Patient in Berührung steht.

Es ist auch bereits bekannt, einen im Wasser liegenden Patienten mit Klängen zu beaufschlagen.

### STAND DER TECHNIK

Das deutsche Gebrauchsmuster DE 20 2010 003 176 U1 betrifft eine Wasserklangmassageliege, die einen beheizten Wasserkern aufweist, auf welchem der Patient druckfrei und entspannt liegt. Unter dem Wasserkern befinden sich Fächer für Klangschalen sowie ein Resonanzraum. Die Klangschalen können von außen angeschlagen werden. Die Schallübertragung erfolgt von den Klangschalen ausgehend durch den Resonanzraum auf die als Wasserbett ausgestaltete Massageliege und von dort auf den Körper eines auf der Massageliege befindlichen Patienten, wobei allerdings die Körperschallübertragung nur über die Berührungsfläche des Patienten mit der Außenhaut der Wasserbett-Liege erfolgt.

Die DE 198 43 379 C2 beschreibt einen multimodalen medizinischen und therapeutisch geschlossenen Badetank, der mit einem Lautsprechersystem für eine Klangtherapie versehen ist. Dazu sind in Ohrhöhe eines Badenden in die Innenwandung des Badetanks Lautsprecher integriert. Eine Schallübertragung über das Wasser erfolgt hier nicht.

Die WO 98/27923 A1 beschreibt ein akustisches Unterhaltungs- und Therapiesystem für Badewannen. Dort sind - ähnlich wie beim vorgenannten Stand der Technik - Lautsprecher oberhalb der Wasserlinie in Ohrhöhe eines Badenden und zusätzlich Unterwasserlautsprecher angebracht.

Die letzten beiden genannten Druckschriften befassen sich mit der Beaufschlagung des Patienten mit künstlich durch Lautsprecher erzeugtem Schall, was den Nachteil aufweist, dass das Frequenzspektrum, welches auf den Patienten einwirkt, insbesondere bei der Verwendung von Unterwasserlautsprechern gegenüber den originalen klangerzeugenden Instrumenten, stark eingeschränkt ist. Zwar ist aus dem erstgenannten Stand der Technik die unmittelbare Klangerzeugung mittels Klangschalen bekannt, die dabei erzeugten Schallwellen werden jedoch durch unterschiedliche Medien (zunächst durch Luft und dann durch Wasser) auf den Patienten übertragen, so dass auch hier eine Filterung des Klangspektrums stattfindet.

Die US 1,164,501 A beschreibt und zeigt ein unter Wasser spielbares Musikinstrument. Dieses Musikinstrument, bei welchem es sich um ein Blasinstrument handelt, ist an einer galgenartigen Aufzugsvorrichtung befestigt, die an ihrem unteren Ende eine Standfläche für eine das Instrument spielende Person aufweist. Diese Person, die auf der Standfläche steht, bläst in das Mundstück des fest am galgenartigen Aufzug angebrachten Blasinstruments, wobei die Person vollständig in das Wasser eingetaucht ist und das Mundstück des Blasinstruments zusammen mit dem unteren Teil des zu einem über der Wasserlinie befindlichen Schalltrichter führenden Blasrohrs ebenfalls in das Wasser eingetaucht ist.

Die US 2009/0223345 A1 betrifft eine mit einem Fluid betriebene Multimediaeinrichtung, wie beispielsweise eine in ein Fluid eintauchbare Multimediaeinrichtung. Das Fluid kann beispielsweise Luft oder Wasser sein. Das Fluid kann von dieser Multimediaeinrichtung angesaugt und ausgestoßen werden. Diese Multimediaeinrichtung kann auch unter Wasser eingesetzt werden, um dann von badenden Personen beispielsweise mit den Füßen betätigt zu werden. Einen schwimmenden Klangkörper weist diese Multimediaeinrichtung nicht auf.

Die DE 34 40 329 A1 zeigt und beschreibt eine Vorrichtung zum Erzeugen von Tönen in einem Gehäuse als bewegbare Einheit. Dabei ist in einem geschlossenen Gehäuse, welches mit einer Mehrzahl von Schallaustrittsöffnungen versehen ist, ein mit einem Lautsprecher versehenes elektronisches Musikwerk enthalten, das über von außen an das Gehäuse gehaltene Magneten mittels eines innerhalb des Gehäuses befindlichen Reed-Kontaktes ein- und ausschaltbar ist. Dieses Musikwerk kann auch schwimmfähig ausgebildet sein. Dabei ist das Gehäuse des Musikwerks an seiner aus dem Wasser herausragenden Seite mit einer Schallaustrittsöffnung versehen.

### DARSTELLUNG DER ERFINDUNG

Aufgabe der vorliegenden Erfindung ist es, eine gattungsgemäße Vorrichtung zum Erzeugen von Schallwellen in Wasser anzugeben, die möglichst original und unverfälscht die Klänge auf einen im Wasser liegenden Patienten, beispielsweise einen menschlichen oder tierischen Körper, überträgt.

Diese Aufgabe wird gelöst durch die Vorrichtung mit den Merkmalen des Patentanspruchs 1. Diese Vorrichtung ist versehen mit zumindest einem im Wasser schwimmenden Klangkörper, der als Musikinstrument ausgebildet oder Teil eines Musikinstruments ist und einen Resonanzkörper aufweist, der an zumindest einer Seite mit dem Wasservolumen zur Schalleinleitung in das Wasser in Kontakt steht, und mit Saiten versehen ist, die bei Anregung den Resonanzkörper und/oder die Luft in einem Luftresonanzraum in Schwingung versetzen.

### VORTEILE

Auf diese Weise werden die erzeugten Schwingungen mittels des schwimmenden Klangkörpers nicht nur an die Luft abgegeben, sondern ebenso an das mit dem Klangkörper in Kontakt stehende Wasser. Dabei wird auch der Klangkörper selbst in Schwingung versetzt. Der mit den Saiten versehene Klangkörper bildet somit ein Saiteninstrument, welches beispielsweise durch Zupfen, Anschlagen der Saiten oder Streichen der Saiten mittels eines Bogens bespielbar ist. Der erzeugte Schall erreicht den Patienten, also den menschlichen oder tierischen Körper, auf zwei Wegen, nämlich einerseits über die Luft und das Gehör des Patienten und andererseits über das Wasser und die im Wasser befindliche Körperoberfläche. Die Übertragung des Schalls durch beide Medien (Luft, Wasser) erfolgt jeweils direkt vom Klangkörper zum Patienten ohne Zwischenschaltung weiterer Medien, so dass das Klangspektrum des durch das jeweilige Medium übertragenen Schalls nicht durch Zwischenschaltung weiterer Medien beeinträchtigt ist.

Bevorzugt weist der Resonanzkörper einen Luftresonanzraum auf.

Vorzugsweise ist der Klangkörper zum Wasservolumen hin von einer Wandung begrenzt. In diesem Fall wird der Klang unmittelbar als Körperschall in das Wasser eingeleitet.

Dabei ist es von Vorteil, wenn die Wandung als Membran ausgebildet ist oder zumindest eine Membran aufweist. Diese Membran verbessert die Klangübertragung aus dem Luftresonanzraum in das Wasser deutlich.

Grundsätzlich kann der Luftresonanzraum auch mit einem Blasinstrument verbunden sein, so dass die vom Blasinstrument zur Schwingung angeregte Luft die Luft im Luftresonanzraum in Schwingung versetzt. Dabei beschränkt sich der Begriff Blasinstrumente nicht nur auf Holzblasinstrumente oder Blechblasinstrumente, sondern bezieht auch andere Instrumente ein, deren Tonerzeugung durch einen Luftstrom erfolgt, wie beispielsweise Orgeln oder Akkordeons.

Selbstverständlich ist die vorliegende Erfindung nicht auf Saiteninstrumente oder Blasinstrumente beschränkt, sondern sie umfasst außerdem auch sogenannte Idiophone, wie beispielsweise Glocken oder Klangschalen, und Membranophone, wie beispielsweise Trommeln oder Pauken.

Eine andere bevorzugte Ausführungsform der Erfindung zeichnet sich dadurch aus, dass der Resonanzkörper bogenförmig ausgebildet ist, dass sich die unteren Enden des Resonanzkörpers auf jeweils einem Schwimmkörper abstützen, wobei der Resonanzkörper mit den Schwimmern zur Körperschallübertragung verbunden ist und so den Klangkörper bildet, und dass die Schwimmkörper seitlich voneinander beabstandet sind, so dass zwischen den Schwimmern und dem bogenförmig gewölbten Resonanzkörper ein Aufenthaltsraum für den schwimmenden menschlichen oder tierischen Körper gebildet ist. Diese Variante ermöglicht die allseitige Beschallung des Patienten.

Eine besonders bevorzugte Weiterbildung der vorliegenden Erfindung zeichnet sich dadurch aus, dass zumindest ein weiterer schwimmender Klangkörper vorgesehen ist, der mit dem ersten Klangkörper über mechanische Verbindungselemente verbindbar oder verbunden ist, und dass die Verbindungselemente zumindest teilweise im Wasser verlaufen und Stützeinrichtungen für den schwimmenden menschlichen oder tierischen Körper bilden. Auf diese Weise wird beispielsweise ein Katamaran oder Trimaran geschaffen, dessen Schwimmkörper von den jeweiligen schwimmenden Klangkörpern gebildet sind. Liegt der Patient zwischen den Klangkörpern derart im Wasser, dass er mit den mechanischen Verbindungselementen der Vorrichtung in Berührung steht, so erfolgt die Klangübertragung vom jeweiligen Klangkörper auf den Patienten nicht nur durch die Luft und über das Wasser, sondern zusätzlich als unmittelbarer Körperschall auch durch die mechanischen Verbindungselemente.

Eine andere vorteilhafte Weiterbildung ist dadurch gekennzeichnet, dass der schwimmende Klangkörper mit externen Klangkörpern mechanisch verbindbar ausgestaltet ist und dazu vorzugsweise zumindest eine Standfläche zum Abstellen von Klangkörpern, zum Beispiel Klangschalen, aufweist. Diese Variante ermöglicht es, mehrere Musikinstrumente zur Schallerzeugung miteinander zu kombinieren. Beispielsweise kann der Klangkörper als Saiteninstrument ausgebildet und zusätzlich mit auf der Standfläche des Klangkörpers abgestellten Idiophonen, beispielsweise Klangschalen, zur Körperschallübertragung verbunden sein. Der von den Klangschalen erzeugte Schall wird damit einerseits über die Luft und andererseits über den schwimmenden Klangkörper und das Wasser zusammen mit den vom Musikinstrument des Klangkörpers unmittelbar erzeugten Schallwellen übertragen.

Eine andere bevorzugte Weiterbildung der Erfindung zeichnet sich dadurch aus, dass der schwimmende Klangkörper einen geschlossenen Luftresonanzraum aufweist, der mit einer ersten, einem Schallerzeuger zugewandten Membran und mit einer zweiten, mit dem Wasservolumen in Verbindung stehenden Membran ausgestattet ist, und dass die erste Membran und die zweite Membran über einen die Membranamplitude verstärkenden Hebelmechanismus derart miteinander verbunden sind, dass die Schwingungsamplitude der zweiten Membran größer ist als die der ersten Membran. Bei dieser Variante wird der von dem zum Klangkörper gehörenden Musikinstrument erzeugte Schall zunächst von der ersten Membran aufgenommen und über den Hebelmechanismus in seiner Amplitude verstärkt, so dass die Amplitude der an das Wasser abgegebenen Schwingung größer ist als die Amplitude, die durch den Schallerzeuger in der ersten Membran hervorgerufen wird.

Eine vorteilhafte Weiterbildung der erfindungsgemäßen Vorrichtung zeichnet sich dadurch aus, dass ein Mikrofon oder ein anderer Schall in elektrische Spannung umwandelnder Schallsensor zur Erzeugung eines die Lautstärke und/oder die Frequenz des aufgenommenen Schalls repräsentierenden elektrischen Signals vorgesehen ist; dass zumindest eine lichterzeugende Einrichtung vorgesehen ist; und dass eine Steuerungseinrichtung vorgesehen ist, der das elektrische Signal zugeleitet wird und die dieses in zumindest ein Lichtsteuerungssignal wandelt, das an die zumindest eine lichterzeugende Einrichtung geleitet wird. Diese Weiterbildung ermöglicht es, dem Patienten nicht nur eine Klangtherapie, sondern gleichzeitig eine Lichttherapie zu verabreichen, wobei die Lichttherapie an die Klangtherapie gekoppelt ist. Der Patient hört also beispielsweise entspannende Musik und wird im Rhythmus der Musik mit Licht von synchron mit dem Klang variierender Farbe und Helligkeit beaufschlagt.

Dabei ist es besonders von Vorteil, wenn das Mikrofon oder der Schallsensor im oder am schwimmenden Klangkörper vorgesehen ist. Diese Anbringung des Schallsensors unmittelbar im oder am schwimmenden Klangkörper sorgt dafür, dass die Ankoppelung der Lichtsteuerung an die Klangerzeugung der Vorrichtung sehr wirksam ist und die Einwirkung von Nebengeräuschen auf die Lichtsteuerung dadurch nahezu ausgeschlossen ist.

Besonders vorteilhaft ist es, wenn das zumindest eine Lichtsteuerungssignal ausgebildet ist, um die Helligkeit und/oder die Farbe des von der zumindest einen lichterzeugenden Einrichtung abgestrahlten Lichts zu beeinflussen. Die hierdurch hervorgerufene Steuerung des von der lichterzeugenden Einrichtung abgegebenen Lichts verschmilzt in der Empfindung des Patienten besonders innig mit dem vom Klangkörper abgegebenen Klang.

Dabei ist bevorzugt die Steuerungseinrichtung so ausgebildet, dass sie die Farbe des von der zumindest einen lichterzeugenden Einrichtung abgestrahlten Lichts in Abhängigkeit von der Klangfrequenz und/oder die Helligkeit des Lichts in Abhängigkeit von der Lautstärke steuert. Hierdurch verschmelzen die Klänge und die Lichteindrücke zu einer die Entspannung besonders fördernde Empfindungssymbiose aus Klang und Licht.

Vorzugsweise ist die Steuerungseinrichtung so ausgebildet, dass sie eine Spektralanalyse des aufgenommenen Schalls durchführt und aufgrund dieser Spektralanalyse zusätzlich zu Hauptklangfrequenz Nebenklangfrequenzen, zum Beispiel Oberwellen, ermittelt und dass aufgrund der Hauptklangfrequenz und zumindest einer Nebenklangfrequenz unterschiedliche lichterzeugende Elemente mit jeweils einem eine Lichtfarbe erzeugenden Lichtsteuerungssignal beaufschlagt werden. Diese Ausführungsform der Erfindung führt zu einem besonders komplexen Klang- und Lichterlebnis für den Patienten.

Besonders vorteilhaft ist es auch, wenn die Vorrichtung mit einer Duftaromen abgebenden Aromatherapieeinrichtung versehen ist. Diese Aromatherapieeinrichtung kann entweder nur mit der Klangerzeugungseinrichtung oder auch mit der Klangerzeugungseinrichtung und der Lichterzeugungseinrichtung gekoppelt sein, so dass dem Patienten neben dem Klangerlebnis und gegebenenfalls dem optischen Erlebnis durch die Lichtbeeinflussung auch ein Dufterlebnis angeboten wird, was sich zusätzlich besonders wirksam auf das gesamtheitliche Wellnessempfinden des Patienten auswirkt.

Bevorzugte Ausführungsbeispiele der Erfindung mit zusätzlichen Ausgestaltungsdetails und weiteren Vorteilen sind nachfolgend unter Bezugnahme auf die beigefügte Zeichnung näher beschrieben und erläutert. In dieser zeigt:
- **Fig. 1**: eine erste Variante der erfindungsgemäßen Vorrichtung;
- **Fig. 2**: eine zweite, gegenüber der Variante der Fig. 1 leicht abgewandelte Variante;
- **Fig. 3**: eine Variante ähnlich der aus Fig. 1, jedoch mit einer zum Wasservolumen weisenden Membran;
- **Fig. 4**: eine "Katamaran"-Variante der erfindungsgemäßen Vorrichtung;
- **Fig. 5**: eine weitere Variante mit einem brückenartigen Resonanzkörper;
- **Fig. 6**: eine Darstellung eines mechanischen Klangverstärkers der erfindungsgemäßen Vorrichtung und
- **Fig. 7**: eine mit einer Lichttherapieeinrichtung und einer Aromatherapieeinrichtung ausgestattete Variante der Vorrichtung am Beispiel der Variante gemäß Fig. 5.

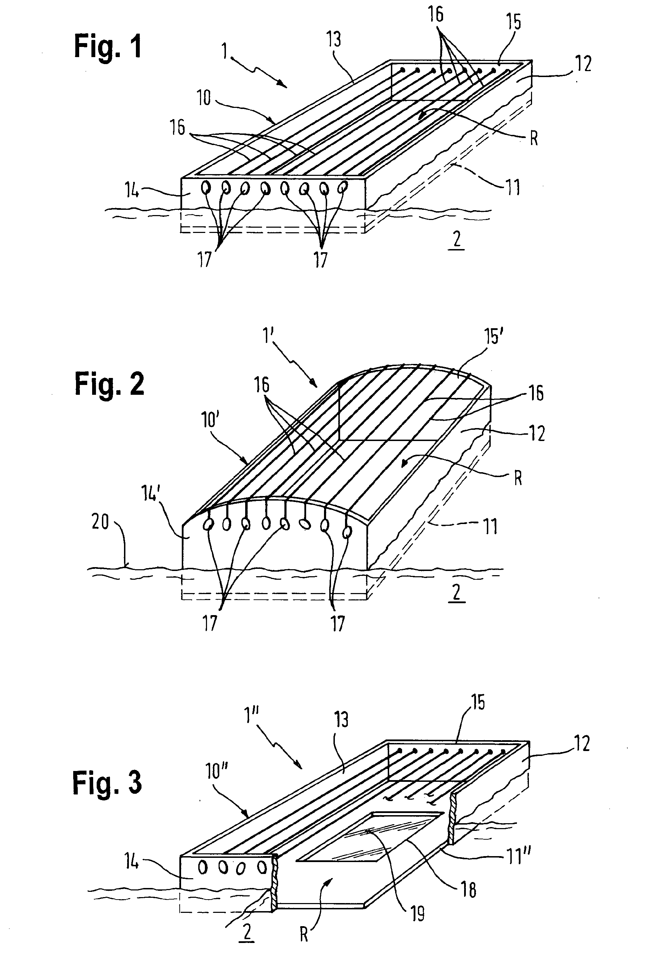

Fig. 1 zeigt eine Vorrichtung zum Erzeugen von Schallwellen in Wasser gemäß der vorliegenden Erfindung. Diese Vorrichtung weist einen im Wasser schwimmenden Klangkörper 1 auf, der als nach oben offener kastenartiger Resonanzkörper 10 mit einer Bodenwand 11, zwei voneinander beabstandeten Längsseitenwänden 12, 13 und zwei Stirnwänden 14, 15 gebildet ist. Diese Vorrichtung kann auch als Klangtherapievorrichtung bezeichnet werden.

Parallel zu den Längsseitenwänden 12, 13 sind mehrere Saiten 16 vorgesehen, die sich zwischen den beiden Stirnwänden 14 und 15 erstrecken und in im Saiteninstrumentenbau üblicherweise mittels Knebeln 17 spannbar sind.

Der Klangkörper 1 ist als Schwimmkörper ausgebildet und daher in der Lage, in einem Wasservolumen 2, beispielsweise einem Schwimmbecken, zu schwimmen. Werden diese Saiten 16 gezupft oder angeschlagen, so geraten der Resonanzkörper 10 und das die Saiten 16 umgebende Luftvolumen, das im Inneren des kastenartigen Resonanzkörpers 10 einen Luftresonanzraum R bildet, in Schwingung. Diese Schwingungen werden einerseits über die Luft übertragen und sind somit akustisch wahrnehmbar und sie werden andererseits durch den Resonanzkörper auf das Wasservolumen 2 übertragen, so dass der beim Bespielen der Saiten 16 entstehende Schall in das Wasser eingeleitet wird und sich dort als Körperschall ausbreitet. Eine im Wasser schwimmende Person kann dann sowohl den Schall hören, als auch über die Haut, die mit dem Wasser in Berührung steht, wahrnehmen. Natürlich ist diese Art der Schallübertragung nicht nur auf die Wahrnehmung durch Menschen beschränkt, sondern kann auch von tierischen Lebewesen wahrgenommen werden.

In Fig. 2 ist eine abgewandelte Ausführungsform der in Fig. 1 gezeigten Vorrichtung dargestellt, bei welcher die Stirnseitenwände 14', 15' an ihrer Oberseite zwischen den beiden Längsseitenwänden 12, 13 eine sich von der Wasseroberfläche 20 weg erstreckende Wölbung aufweisen. Die Saiten 16 verlaufen hier von den seitlich angebrachten Knebeln 17 über die obere gewölbte Kante der jeweiligen Stirnwand 14', 15'. Diese Ausführungsform hat gegenüber der in Fig. 1 gezeigten Ausführungsform den Vorteil, dass sie mittels eines Bogens bespielbar ist, ähnlich wie dies bei einer Geige der Fall ist.

In Fig. 3 ist eine gegenüber der Fig. 1 abgewandelte Variante eines Klangkörpers 1" gezeigt, bei welchem die Bodenwand 11" des Resonanzkörpers 10" mit einer Ausnehmung 18 versehen ist, die von einer Membran 19 wasserdicht verschlossen ist. Diese Membran, die wesentich dünner ist als die Bodenwand 11" und aus einem elastischen Material besteht, ist in der Lage, die Schwingungen der Luft im Luftresonanzraum R auf das Wasser im Wasservolumen 2 zu übertragen.

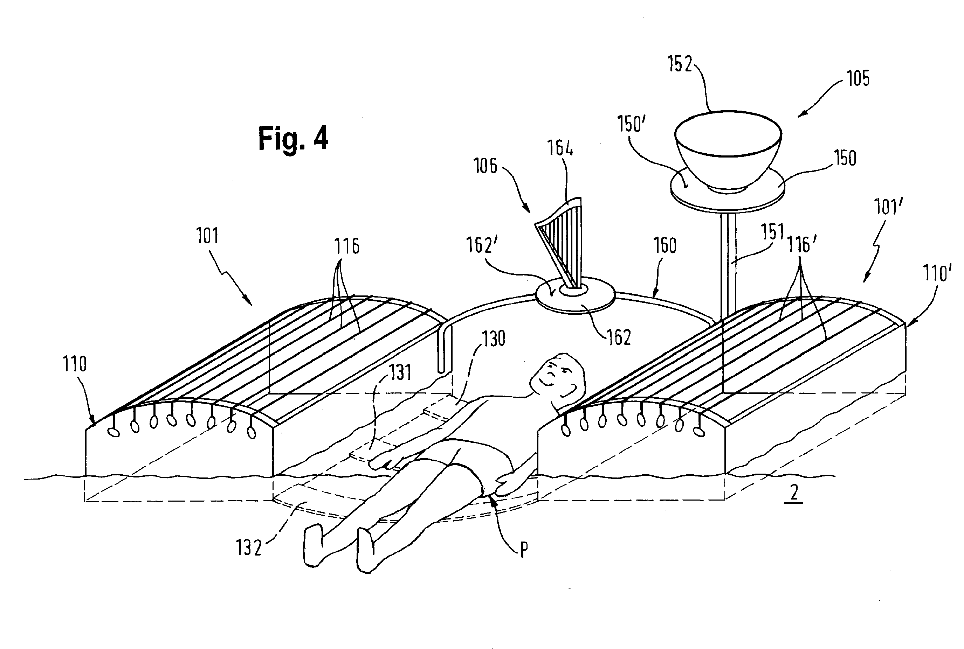

Fig. 4 zeigt eine weitere Ausführungsform der erfindungsgemäßen Vorrichtung, bei welcher zwei entsprechend dem Beispiel der Fig. 2 ausgebildete schwimmende Klangkörper 101, 101' vorgesehen sind, die über drei mechanische Verbindungselemente 130, 131, 132 nach Art eines Katamarans miteinander verbunden sind. Diese Verbindungselemente 130, 131, 132 verlaufen unter Wasser und bilden Stützeinrichtungen für einen im Wasser liegenden Patienten P, der hier als menschlicher Körper dargestellt ist, der aber auch ein tierischer Körper sein kann.

Bei der in Fig. 4 gezeigten Ausführungsform werden die mittels der Saiten 116, 116' der Klangkörper 101, 101' erzeugten akustischen Schwingungen zusätzlich zur bereits geschilderten Übertragung durch die Luft und durch das Wasser auch vom jeweiligen Resonanzkörper 110, 110' und die mit dem jeweiligen Resonanzkörper 110, 110' fest verbundenen mechanischen Verbindungselemente 130, 131, 132 als Körperschall auf den Patienten P übertragen, der im Wasser des Wasservorrats 2 auf den Verbindungselementen 130, 131, 132 liegt.

In Fig. 4 sind beispielshaft auch Zusatzausstattungen für externe Klangkörper 105, 106 gezeigt, die mit jedem der erfindungsgemäßen Klangkörper verbunden sein können.

So ist am Klangkörper 101' eine Stütze 151 mit einem darauf angebrachten, eine Standfläche 150' für eine Klangschale 152 bildenen Teller 150 befestigt. Die von der Klangschale 152 ausgehenden Schwingungen werden als Körperschall auf den Klangkörper 101' und von diesem auf den Patienten P über das Wasser und über die Verbindungselemente 130, 131, 132 übertragen.

Des Weiteren ist in Fig. 4 eine die Klangkörper 101 und 101' mechanisch verbindende Brücke 160 gezeigt, die sich über dem Patienten P erstreckt. Auf der Brücke 160 ist ein Teller 162 mit einer Standfläche 162' angeordnet, auf der beispielsweise eine Luftharfe 164 anbringbar ist. Auch hier wird der Körperschall vom Teller 162 über die Brücke 160 in die Klangkörper 101, 101' eingeleitet.

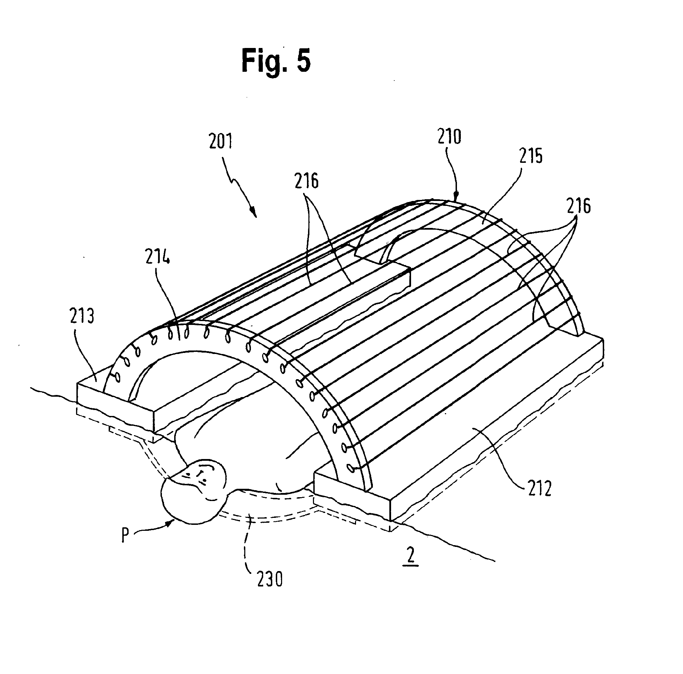

In Fig. 5 ist eine abgewandelte Variante einer ebenfalls katamaranartig ausgebildeten erfindungsgemäßen Vorrichtung dargestellt, bei welcher der Resonanzkörper 210 bogenförmig ausgestaltet ist und die Form eines beispielsweise zylindrischen Rohrsegments mit einer jeweiligen bogenförmigen Stirnwand 214, 215 aufweist. Der Resonanzkörper 210 kann, wie dargestellt, ohne Bodenwand ausgebildet sein, er kann aber auch, wie in den Beispielen der Figuren 1 bis 4, eine Bodenwand aufweisen, die dann entlang der Unterseiten der bogenförmigen Stirnwände 214, 215 bogenförmig verläuft. Die Längsseitenbegrenzungen des bogenförmigen Resonanzkörpers 210 sind jeweils von einem Schwimmkörper 212, 213 gebildet, die beide auf dem Wasser des Wasservorrats 2 schwimmend den Resonanzkörper 210 tragen und gemeinsam mit diesem den Klangkörper 201 bilden.

Die Saiten 216 dieses Klangkörpers 201 sind in der gleichen Weise über die jeweilige bogenförmige Stirnwand 214, 215 gespannt wie dies in Verbindung mit der Fig. 2 beschrieben worden ist, so dass diese Saiten auch mit einem Bogen bespielbar sind.

Unter Wasser erstrecken sich zwischen den beiden Schwimmkörpern 212, 213 drei mechanische Verbindungselemente, von denen nur das den Kopf des Patienten P stützende Verbindungselement 230 gezeigt ist, wie dies in Verbindung mit der Fig. 4 beschrieben worden ist. Der Patient P liegt zwischen den Schwimmkörpern 212, 213 im Wasser unterhalb es Resonanzkörpers 210, wobei der Körper des Patienten P auf den Verbindungselementen abgestützt ist.

Auch bei der Ausführungsform der Fig. 5 erfolgt die Schallübertragung über die Luft, über das Wasser und als Körperschall über die mechanischen Verbindungselemente zwischen den Schwimmkörpern 212, 213.

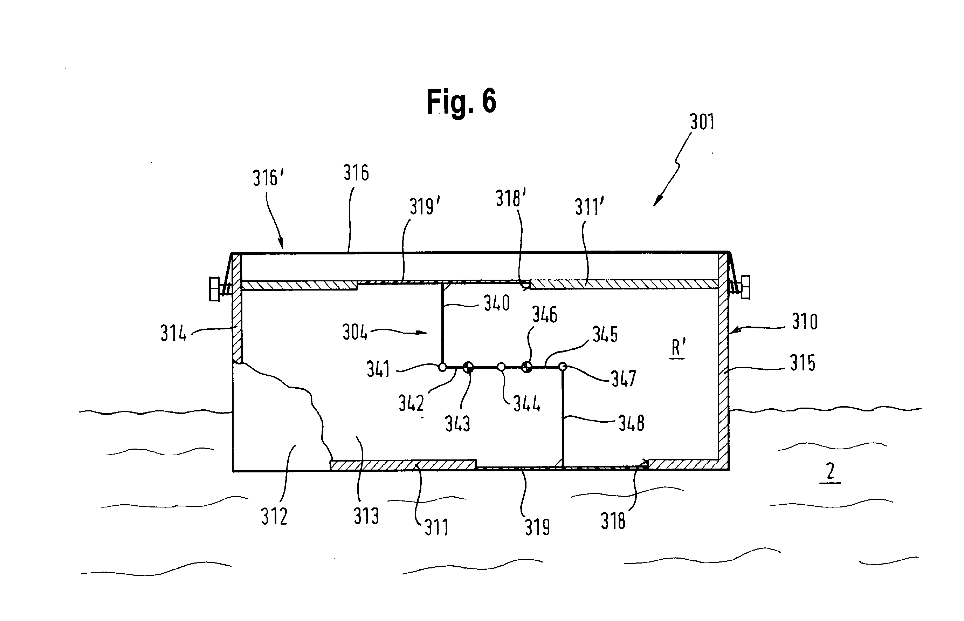

Fig. 6 zeigt einen Längsschnitt durch eine abgewandelte Variante der erfindungsgemäßen Vorrichtung mit einem Klangkörper 301, der einen geschlossenen Resonanzkörper 310 aufweist. Dieser geschlossene Resonanzkörper 310 schließt einen nach außen abgeschlossenen Luftresonanzraum R' ein. Auch hier ist der Resonanzkörper 310 als Kasten mit zwei Längsseitenwänden 312, 313 sowie zwei Stirnwänden 314, 315 und einer Bodenwand 311 ausgebildet. Von der Bodenwand 311 nach oben, in Richtung auf die Saiten 316 hin, beabstandet ist eine zur Bodenwand 311 parallele Zwischenwand 311' vorgesehen, die sich zwischen den Längsseitenwänden 312, 313 und den Stirnwänden 314, 315 erstreckt und die den Luftresonanzraum R' nach oben hin abschließt.

Auch die obere Zwischenwand 311' ist mit einer Ausnehmung 318' versehen, welche ihrerseits von einer Membran 319' verschlossen ist. Zwischen den beiden Membranen 319, 319' erstreckt sich ein Hebelmechanismus 304, der die Membranen 319, 319' mechanisch miteinander verbindet.

Der Hebelmechanismus 304 weist einen mit der oberen, ersten Membran 319' verbundenen ersten Membranstößel 340 auf, der mit einem ersten Schwenkhebel 342 über ein Gelenk 341 verbunden ist. Der erste Schwenkhebel 342 ist mittels eines Schwenkgelenks 343 am Gehäuse des Resonanzkörpers 310 schwenkbar gelagert. Das vom Gelenk 341 abgewandte Ende des ersten Schwenkhebels 342 ist über ein weiteres Gelenk 344 mit einem zweiten Schwenkhebel 345 gelenkig verbunden, an dessen anderem Ende ein zweiter, mit der unteren zweiten Membran 319 fest verbundener Membranstößel 348 mittels eines Gelenks 347 gelenkig gelagert ist. Auch der zweite Schwenkhebel 345 ist wie der erste Schwenkhebel 342 mittels eines Schwenkgelenks 346 am Gehäuse des Resonanzkörpers 310 schwenkbar gelagert.

Die Dimensionen der einzelnen Elemente des Hebelmechanismus' 304 sind so bemessen, dass eine Vertikalbewegung des ersten Membranstößels 340 eine Vertikalbewegung des zweiten Membranstößels 348 um eine größere Strecke bewirkt. Dadurch ist die Schwingungsamplitude der zweiten Membran 319 größer als die Schwingungsamplitude der ersten Membran, was dazu führt, dass eine von dem durch die Saiten 316 gebildeten Schallerzeuger 316' auf die obere, erste Membran 319' induzierte Schwingung von der unteren, zweiten Membran 319 mit größerer Amplitude, also verstärkt, auf das Wasser des Wasservorrats 2 übertragen wird.

Die in Fig. 1 bis Fig. 5 gezeigten Varianten mit nach oben offenem Resonanzraum R können alternativ, ähnlich wie im Beispiel der Fig. 6, auch mit einer oberen Zwischenwand ausgestattet sein, wobei diese obere Zwischenwand geschlossen ausgebildet, mit Schallöffnungen ausgestattet oder mit einer Membran versehen sein kann.

Grundsätzlich ist es auch möglich, in den schwimmenden Klangkörpern zusätzlich zu den Saiten Lautsprecher vorzusehen, die mit Tonwiedergabegeräten verbunden sind.

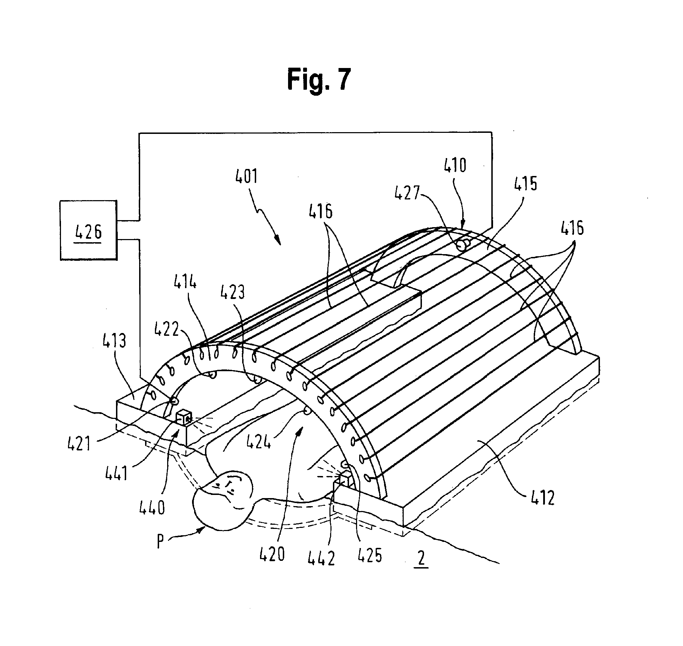

In Fig. 7 ist eine weitere abgewandelte Variante eines katamaranartig ausgebildeten erfindungsgemäßen Klangkörpers 401 dargestellt, der prinzipiell der Variante aus Fig. 5 entspricht. Zusätzlich sind hier eine Lichttherapieeinrichtung 420 und eine Aromatherapieeinrichtung 440 vorgesehen. An zumindest einer der bogenförmigen Stirnwände 414, 415 des bogenförmig ausgestalteten Resonanzkörpers 410 ist eine Reihe von lichterzeugenden Einrichtungen 421, 422, 423, 424, 425, beispielsweise LED-Leuchten, vorgesehen, die von einer nur schematisch dargestellten Steuerungseinrichtung 426 jeweils mit elektrischer Energie versorgt werden. Ein am Klangkörper 401 angebrachtes Mikrofon 427 nimmt den vom Klangkörper 401 und von dessen Saiten 416 abgestrahlten Schall auf und leitet ein diesen repräsentierendes elektrisches Signal an die Steuerungseinrichtung 426 weiter. Die Steuerungseinrichtung 426 setzt dieses empfangene elektrische Signal, beispielsweise wie bei einer Lichtorgel, in zumindest ein Lichtsteuerungssignal um, das an eine oder mehrere der lichterzeugenden Einrichtungen 421, 422, 423, 424, 425 geleitet wird. Die vom Mikrofon 427 aufgenommene Klangfrequenz bestimmt dabei die Lichtfarbe und die Lautstärke des aufgenommenen Schalls bestimmt die Helligkeit des abgestrahlten Lichts.

Weiterhin sind im Beispiel der Fig. 7 zwei Duftspender 441, 442 der Aromatherapieeinrichtung 440 vorgesehen, die jeweils auf einem der Schwimmkörper 412, 413 in der Nähe der kopfseitigen bogenförmigen Stirnwand 414 angebracht sind.

Die Ausgestaltung der erfindungsgemäßen Vorrichtung mit einer Lichttherapieeinrichtung und/oder einer Aromatherapieeinrichtung ist vorstehend zwar am Ausführungsbeispiel der Fig. 5 und 7 gezeigt und beschrieben worden, doch ist es ebenfalls von der Erfindung umfasst, die in den Fig. 1 bis 4 gezeigten Varianten einer erfindungsgemäßen Klangtherapievorrichtung mit einer Lichttherapieeinrichtung und/oder einer Aromatherapieeinrichtung auszustatten.

Die Erfindung ist nicht auf das obige Ausführungsbeispiel beschränkt, das lediglich der allgemeinen Erläuterung des Kerngedankens der Erfindung dient. Im Rahmen des Schutzumfangs kann die erfindungsgemäße Vorrichtung vielmehr auch andere als die oben beschriebenen Ausgestaltungsformen annehmen. Die Vorrichtung kann hierbei insbesondere Merkmale aufweisen, die eine Kombination aus den jeweiligen Einzelmerkmalen der Ansprüche darstellen.

Bezugszeichen in den Ansprüchen, der Beschreibung und den Zeichnungen dienen lediglich dem besseren Verständnis der Erfindung und sollen den Schutzumfang nicht einschränken.

### Bezugszeichenliste

- 1: Klangkörper
- 1": Klangkörper
- 2: Wasservolumen
- 10: Resonanzkörper
- 10": Resonanzkörper
- 11: Bodenwand
- 11": Bodenwand
- 12: Längsseitenwand
- 13: Längsseitenwand
- 14: Stirnseitenwand
- 14': Stirnseitenwand
- 15: Stirnseitenwand
- 15': Stirnseitenwand
- 16: Saite
- 17: Knebel
- 18: Ausnehmung
- 19: Membran
- 20: Wasseroberfläche
- 101: Klangkörper
- 101': Klangkörper
- 105: externer Klangkörper
- 106: externer Klangkörper
- 110: Resonanzkörper
- 110': Resonanzkörper
- 130: Verbindungselement
- 131: Verbindungselement
- 132: Verbindungselement
- 150: Teller
- 150': Standfläche
- 151: Stütze
- 152: Klangschale
- 160: Brücke
- 162: Teller
- 162': Standfläche
- 164: Luftharfe
- 201: Klangkörper
- 210: Resonanzkörper
- 212: Schwimmkörper
- 213: Schwimmkörper
- 214: Stirnwand
- 215: Stirnwand
- 216: Saiten
- 230: Verbindungselment
- 301: Klangkörper
- 304: Hebelmechanismus
- 310: Resonanzkörper
- 311: Bodenwand
- 311': obere Zwischenwand
- 312: Längsseitenwand
- 313: Längsseitenwand
- 314: Stirnwand
- 315: Stirnwand
- 316: Saite
- 318': Ausnehmung
- 319: Membran
- 319': Membran
- 340: Membranstößel
- 341: Gelenk
- 342: Schwenkhebel
- 344: Gelenk
- 345: Schwenkhebel
- 346: Schwenkgelenk
- 347: Gelenk
- 348: Membranstößel
- 401: Klangkörper
- 410: Resonanzkörper
- 412: Schwimmkörper
- 413: Schwimmkörper
- 414: bogenförmige Stirnwand
- 415: bogenförmige Stirnwand
- 416: Saite
- 420: Lichttherapieeinrichtung
- 421: lichterzeugende Einrichtung
- 422: lichterzeugende Einrichtung
- 423: lichterzeugende Einrichtung
- 424: lichterzeugende Einrichtung
- 425: lichterzeugende Einrichtung
- 426: Steuerungseinrichtung
- 427: Mikrofon
- 440: Aromatherapieeinrichtung
- 441: Duftspender
- 442: Duftspender

- P: Patient
- R: Luftresonanzraum

## Patentansprüche

1. Vorrichtung zum Erzeugen von Schallwellen in Wasser, insbesondere zum Erzeugen von multimedial auf einen in einem Wasservolumen befindlichen menschlichen oder tierischen Körper (P) einwirkenden Schallwellen, mit zumindest einem im Wasser schwimmenden Klangkörper (1, 1', 1", 101, 101', 201, 301),
**dadurch gekennzeichnet,**
**dass** der schwimmende Klangkörper (1, 1', 1", 101, 101', 201, 301) als Musikinstrument ausgebildet oder der Teil eines Musikinstruments ist und einen Resonanzkörper (10, 10', 10", 110, 110', 210, 310) aufweist, der an zumindest einer Seite mit dem Wasservolumen (2) zur Schalleinleitung in das Wasser in Kontakt steht, und
**dass** der Klangkörper mit Saiten (16, 116, 116', 216, 316) versehen ist, die bei Anregung den Resonanzkörper (10, 10', 10", 110, 110', 210, 310) und/oder die Luft in einem Luftresonanzraum (R, R') in Schwingung versetzen.

2. Vorrichtung nach Anspruch 1,
**dadurch gekennzeichnet,**
**dass** der Resonanzkörper (10, 10', 10", 110, 110', 210, 310) einen Luftresonanzraum (R, R') aufweist.

3. Vorrichtung nach Anspruch 1 oder 2,
**dadurch gekennzeichnet,**
**dass** der Klangkörper (1, 1', 1", 101, 101', 201, 301) zum Wasservolumen (2) hin von einer Wandung (11, 11 ", 311) begrenzt ist.

4. Vorrichtung nach Anspruch 3,
**dadurch gekennzeichnet,**
**dass** die Wandung (11, 11 ", 311) als Membran ausgebildet ist oder eine Membran (19, 319) aufweist.

5. Vorrichtung nach einem der vorhergehenden Ansprüche,
**dadurch gekennzeichnet,**
**dass** zumindest ein weiterer schwimmender Klangkörper (101') vorgesehen ist, der mit dem ersten Klangkörper (101) über mechanische Verbindungselemente (130, 131, 132) verbindbar oder verbunden ist, und dass die Verbindungselemente (130, 131, 132) zumindest teilweise im Wasser verlaufen und Stützeinrichtungen für den schwimmenden menschlichen oder tierischen Körper (P) bilden.

6. Vorrichtung nach einem der vorhergehenden Ansprüche,
**dadurch gekennzeichnet,**
- **dass** der Resonanzkörper (210) bogenförmig ausgebildet ist,
- **dass** sich die unteren Enden des Resonanzkörpers (210) auf jeweils einem Schwimmkörper (212, 213) abstützen, wobei der Resonanzkörper (210) mit den Schwimmkörpern (212, 213) zur Körperschallübertragung verbunden ist und so den Klangkörper (201) bildet, und
- **dass** die Schwimmkörper (212, 213) seitlich voneinander beabstandet sind, so dass zwischen den Schwimmkörpern (212, 213) und dem bogenförmig gewölbten Resonanzkörper (210) ein Aufenthaltsraum für den schwimmenden menschlichen oder tierischen Körper (P) gebildet ist.

7. Vorrichtung nach einem der vorhergehenden Ansprüche,
**dadurch gekennzeichnet,**
**dass** der schwimmende Klangkörper (101') mit externen Klangkörpern (105, 106) mechanisch verbindbar ausgestaltet ist und dazu vorzugsweise zumindest eine Standfläche (150) zum Abstellen von Klangkörpern (105, 106), zum Beispiel Klangschalen (152), aufweist.

8. Vorrichtung nach einem der vorhergehenden Ansprüche,
**dadurch gekennzeichnet,**
**dass** der schwimmende Klangkörper (301) einen geschlossenen Luftresonanzraum (R') aufweist, der mit einer ersten, einem Schallerzeuger (316') zugewandten Membran (319') und mit einer zweiten, mit dem Wasservolumen (2) in Verbindung stehenden Membran (319) ausgestattet ist, und dass die erste Membran (319') und die zweite Membran (319) über einen die Membranamplitude verstärkenden Hebelmechanismus (304) derart miteinander verbunden sind, dass die Schwingungsamplitude der zweiten Membran (319) größer ist als die der ersten Membran (319').

9. Vorrichtung nach einem der vorhergehenden Ansprüche,
**dadurch gekennzeichnet,**
- **dass** ein Mikrofon oder ein anderer Schall in elektrische Spannung umwandelnder Schallsensor zur Erzeugung eines die Lautstärke und/oder die Frequenz des aufgenommenen Schalls repräsentierenden elektrischen Signals vorgesehen ist;
- **dass** zumindest eine lichterzeugende Einrichtung vorgesehen ist; und
- **dass** eine Steuerungseinrichtung vorgesehen ist, der das elektrische Signal zugeleitet wird und die dieses in zumindest ein Lichtsteuerungssignal wandelt, das an die zumindest eine lichterzeugende Einrichtung geleitet wird.

10. Vorrichtung nach Anspruch 9,
**dadurch gekennzeichnet,**
**dass** das Mikrofon oder der Schallsensor im oder am schwimmenden Klangkörper vorgesehen ist.

11. Vorrichtung nach Anspruch 9 oder 10,
**dadurch gekennzeichnet,**
**dass** das zumindest eine Lichtsteuerungssignal ausgebildet ist, um die Helligkeit und/oder die Farbe des von der zumindest einen lichterzeugenden Einrichtung abgestrahlten Lichts zu beeinflussen.

12. Vorrichtung nach Anspruch 11,
**dadurch gekennzeichnet,**
**dass** die Steuerungseinrichtung so ausgebildet ist, dass sie die Farbe des von der zumindest einen lichterzeugenden Einrichtung abgestrahlten Lichts in Abhängigkeit von der Klangfrequenz und/oder die Helligkeit des Lichts in Abhängigkeit von der Lautstärke steuert.

13. Vorrichtung nach Anspruch 12,
**dadurch gekennzeichnet,**
**dass** die Steuerungseinrichtung so ausgebildet ist, dass sie eine Spektralanalyse des aufgenommenen Schalls durchführt und aufgrund dieser Spektralanalyse zusätzlich zu Hauptklangfrequenz Nebenklangfrequenzen, zum Beispiel Oberwellen, ermittelt und dass aufgrund der Hauptklangfrequenz und zumindest einer Nebenklangfrequenz unterschiedliche lichterzeugende Elemente mit jeweils einem eine Lichtfarbe erzeugenden Lichtsteuerungssignal beaufschlagt werden.

14. Vorrichtung nach einem der vorhergehenden Ansprüche,
**dadurch gekennzeichnet,**
**dass** die Vorrichtung mit einer Duftaromen abgebenden Aromatherapieeinrichtung versehen ist.
